# EUROPEAN PATENT SPECIFICATION

(11) **EP 3 365 060 B1**
(45) Date of publication and mention of the grant of the patent: **25.03.2020**
(21) Application number: 16790293.1
(22) Date of filing: 18.10.2016
(51) Int. Cl.: A61N 7/00, A61N 7/02, A61N 5/04, A61N 1/40, A61M 25/01, A61B 34/10, A61B 34/20, A61N 5/10

(54) **SYSTEM SUPPORTING TREATMENT OF A SUBJECT**
SYSTEM ZUR UNTERSTÜTZUNG DER BEHANDLUNG EINER PERSON
SYSTÈME PRENANT EN CHARGE LE TRAITEMENT D'UN SUJET

(30) Priority: 21.10.2015 EP 15190834
(43) Date of publication of application: 29.08.2018
(73) Proprietor: Koninklijke Philips N.V., 5656 AG Eindhoven (NL)
(72) Inventor: ELEVELT, Aaldert, Jan, 5656 AE Eindhoven (NL); DONATO, Katia, 5656 AE Eindhoven (NL); REM-BRONNEBERG, Debbie, 5656 AE Eindhoven (NL); SHULEPOV, Sergei, 5656 AE Eindhoven (NL); BARAGONA, Marco, 5656 AE Eindhoven (NL); HAUTVAST, Guillaume, Leopold, Theodorus, Frederik, 5656 AE Eindhoven (NL); MAESSEN, Ralph, Theodorus, Hubertus, 5656 AE Eindhoven (NL); DI TULLIO, Alessandra, 5656 AE Eindhoven (NL)
(74) Representative: Philips Intellectual Property & Standards
(86) International application number: PCT/EP2016/074916
(87) International publication number: WO 2017/067897

(56) References cited:
- EP-A1- 1 481 637
- WO-A1-2013/130895
- WO-A2-2015/011690
- US-A1- 2006 122 514
- US-A1- 2008 287 860

## Description

### FIELD OF THE INVENTION

The invention relates to a system and method for planning a treatment of a subject. The invention relates further to a computer program for controlling the system in accordance with the method for planning the treatment of the subject. Moreover, the invention relates to an arranging device for arranging a treatment device.

### BACKGROUND OF THE INVENTION

In interstitial tumor therapy needles are arranged within or close to a tumor. The needles are adapted to deliver energy to the tumor and to the region surrounding the tumor, wherein the energy is chosen such that the tumor is ablated. It is difficult to arrange the needles accurately enough such that substantially only the tumor is ablated and no surrounding healthy tissue, which can lead to unwanted therapy side effects.

### SUMMARY OF THE INVENTION

It is an object of the present invention to provide a system and method for planning a treatment of a subject, which allows for a treatment with reduced therapy side effects. It is a further object of the present invention to provide a corresponding computer program and an arranging device for arranging a treatment device.

In a first aspect of the present invention a system for planning a treatment of a subject is presented, wherein the system is adapted to plan a treatment to be carried out by using a treatment device, wherein the treatment device is elongated and comprises a longitudinal axis and an energy delivery element which does not completely encircle the treatment device and which is adapted to deliver energy to be used for treating the subject, wherein the system comprises:
a treatment planning device for planning the treatment of the subject, wherein the treatment planning device is adapted to determine a treatment position of the treatment device to be used during the treatment in a four-dimensional space, wherein the four-dimensional space is representable by three Cartesian coordinates and an angular coordinate, wherein the angular coordinate defines the angular orientation of the treatment device with respect to a rotation around its longitudinal axis.

Since the energy delivery element does not completely encircle the elongated treatment device, the energy delivery is a directional energy delivery, wherein the energy may be directed to one or several directions. Preferentially, the energy delivery element is adapted to deliver the energy in a single direction only, i.e. the treatment device is preferentially a unidirectional treatment device. Moreover, since the treatment planning device does not only determine the three-dimensional position of the treatment device, which should be used during the treatment, but also a rotational position of the treatment device with respect to a rotation of the treatment device around its longitudinal axis, the energy delivery can be very accurately planned, which allows for a treatment with reduced unwanted therapy side effects.

The elongated treatment device is, for instance, a needle or a catheter, especially a micro catheter. Moreover, the treatment device is preferentially an interstitial treatment device. The energy delivery element is preferentially adapted to deliver thermal energy. However, it can also be adapted to deliver another kind of energy. In an embodiment the energy delivery element is a high intensity focused ultrasound (HIFU) element.

The treatment planning device can be adapted to determine the position of the treatment device and the amount of energy to be delivered at the determined position based on an image data set, which shows at least a region to be treated, and based on known relations between a) the shape and extension of ablation regions and b) the amount of energy delivered by the energy delivery element, wherein the ablation region defines a region around the treatment device, in which tissue will be ablated, given the respective amount of energy delivered by the energy delivery element. The treatment planning device is preferentially adapted to plan the treatment of the subject such that substantially only the tumor and optionally also a safety margin around the tumor are within the ablation region, but substantially no further surrounding tissue.

In an embodiment the system further comprises an arranging device for receiving the treatment position determined by the treatment planning device and for arranging the treatment device in accordance with the received treatment position. The arranging device can be a unit that is adapted to assist a user in arranging the treatment device in accordance with the determined treatment position. For instance, the arranging device can be adapted to determine a current position of the treatment device and to output a deviation of the current position from the planned treatment position. In particular, the arranging device can be adapted to show the planned treatment position of the treatment device and the current position of the treatment device on a display, in order to visualize the deviation, wherein the user can modify the current position of the treatment device such that it finally corresponds to the planned treatment position. However, the arranging device can also be adapted to directly arrange the treatment device in accordance with the planned treatment position. For instance, the arranging device can comprise a robotic device for automatically arranging the treatment device in accordance with the planned treatment position.

The treatment planning device can be adapted to plan a treatment of the subject with a single treatment device only or with several treatment devices, wherein in the latter case preferentially each treatment device is elongated and comprises a longitudinal axis and an energy delivery element which does not completely encircle the respective treatment device. Correspondingly, also the arranging device can be adapted for arranging a single treatment device only or several treatment devices in accordance with the respective determined treatment positions.

The arranging device may include a support structure comprising at least one opening for receiving the treating device, wherein the support structure may be adapted such that the treatment device is rotatable relative to the support structure, in order to arrange the treatment device in accordance with the angular coordinate of the treatment position. The support structure may comprise at least one holding element comprising the at least one opening for receiving the treatment device, wherein the holding element may be rotatable relative to the support structure, in order to arrange the treatment device in accordance with the angular coordinate of the treatment position. Moreover, the at least one opening may have a non-circular cross section. Preferentially, the support structure comprises several openings for receiving the treatment device, wherein the openings are arranged in a holding plane and are adapted such that the treatment device is movable in a direction being perpendicular to the holding plane. By using this support structure the accuracy of arranging the treatment device in accordance with the determined treatment position can be improved, thereby further decreasing the likelihood of unwanted therapy side effects.

The arranging device may comprise a six-degrees-of-freedom sensor for determining the position of the treatment device, wherein the six-degrees-of-freedom sensor is preferentially an electromagnetic sensor. By using the six-degrees-of-freedom sensor the arranging device can accurately determine the current position of the treatment device. This information can be used, for instance, for assisting a user while arranging the treatment device in accordance with the planned treatment position. The accurately determined current position of the treatment device may also be used by an optional robotic device of the arranging device, in order to improve the accuracy of arranging the treatment device in accordance with the planned treatment position.

The six-degrees-of-freedom sensor may comprise at least two location sensors arranged at opposite sides of the energy delivery element. For instance, a first electromagnetic sensor can be placed adjacent to a first side of the energy delivery element and a second electromagnetic sensor can be placed adjacent to a second, opposite side of the energy delivery element. However, it is also possible that the six-degrees-of-freedom sensor comprises at least two location sensors arranged with a distance to the energy delivery element. By placing the at least two location sensors with a distance to the energy delivery element unwanted interferences between a) energy delivery and b) determining the current position of the treatment device can be reduced or even eliminated. This can also lead to an improved accuracy of arranging the treatment device in accordance with the planned treatment position and can further reduce unwanted therapy side effects.

The energy delivery element preferentially comprises a flat surface emitting the energy. If a flat energy delivery surface is used, the energy is delivered in a single direction only being perpendicular to the energy delivery surface. This can lead to a more focused energy delivery, which in turn can lead to a further reduced likelihood of unwanted therapy side effects.

In a further aspect of the present invention an arranging device for arranging a treatment device is presented, the treatment device being elongated and comprising a longitudinal axis and an energy delivery element which does not completely encircle the treatment device and which is adapted to deliver energy to be used for treating the subject, the arranging device being adapted for receiving a treatment position determined by a treatment planning device of a system as defined in claim 1 and for arranging the treatment device in accordance with the received treatment position.

The invention relates also to a method for treating a subject, wherein the method is adapted to treat the subject by using a treatment device, wherein the treatment device is elongated and comprises a longitudinal axis and an energy delivery element which does not completely encircle the treatment device and which is adapted to deliver energy to be used for treating the subject, wherein the method comprises:
- planning a treatment of a subject by using a treatment planning device, wherein a treatment position of the treatment device to be used during the treatment is determined in a four-dimensional space, wherein the four-dimensional space is representable by three Cartesian coordinates and an angular coordinate, wherein the angular coordinate defines the angular orientation of the treatment device with respect to a rotation around its longitudinal axis,
- arranging the treatment device in accordance with the treatment position.
In a further aspect of the present invention a method for planning a treatment of a subject is presented, the method being adapted to plan a treatment to be carried out by using a treatment device, the treatment device being elongated and comprising a longitudinal axis and an energy delivery element which does not completely encircle the treatment device, the method comprising:
- planning the treatment of the subject by using a treatment planning device, wherein a treatment position of the treatment device to be used during the treatment is determined in a four-dimensional space, wherein the four-dimensional space is representable by three Cartesian coordinates and an angular coordinate, wherein the angular coordinate defines the angular orientation of the treatment device with respect to a rotation around its longitudinal axis.

The invention relates also to a method for arranging a treatment device, the treatment device being elongated and comprising a longitudinal axis and an energy delivery element which does not completely encircle the treatment device, the method comprising:
- receiving a treatment position determined by a treatment planning device of a system as defined in claim 1 by an arranging device, and
- arranging the treatment device in accordance with the received treatment position.

The invention relates also to a computer program for controlling a system for treating a subject, wherein the computer program comprises program code means for causing the system to carry out the steps of the method for treating a subject, when the computer program is run on a computer controlling the system.

In a further aspect of the present invention a computer program for controlling a system for planning a treatment of a subject as defined in claim 1 is presented, wherein the computer program comprises program code means for causing the system to carry out the steps of the method for planning a treatment of a subject as defined in claim 14, when the computer program is run on a computer controlling the system.

The invention relates also to a computer program for controlling an arranging device as defined in claim 13, wherein the computer program comprises program code means for causing the arranging device to carry out the steps of the method for arranging a treatment device, when the computer program is run on a computer controlling the arranging device.

It shall be understood that the system of claim 1, the arranging device of claim 13, the method for treating a subject, the method of claim 14, the method for arranging a treatment device, the computer program for controlling a system for treating a subject, the computer program of claim 15 and the computer program for controlling an arranging device have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a system for treating a subject,
Fig. 2 shows schematically and exemplarily an embodiment of a placing unit for placing needles within a prostate of the subject,
Fig. 3 shows schematically and exemplarily a supporting structure of the placing unit shown in Fig. 2,
Fig. 4 shows schematically and exemplarily a cross section of an embodiment of a needle,
Fig. 5 shows schematically and exemplarily an opening of the support structure shown in Fig. 3,
Fig. 6 shows schematically and exemplarily a further embodiment of a system for treating a subject,
Fig. 7 shows schematically and exemplarily a further embodiment of a needle,
Fig. 8 shows schematically and exemplarily a cross section of the needle shown in Fig. 7,
Fig. 9 shows schematically and exemplarily a further embodiment of a needle,
Fig. 10 schematically illustrates a result of a treatment planning procedure,
Fig. 11 shows a flowchart exemplarily illustrating an embodiment of a method for treating a subject, and
Fig. 12 illustrates schematically several elements shown in an overlay view on a display.

### DETAILED DESCRIPTION OF EMBODIMENTS

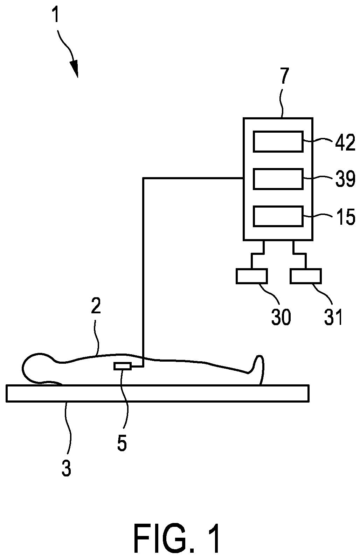

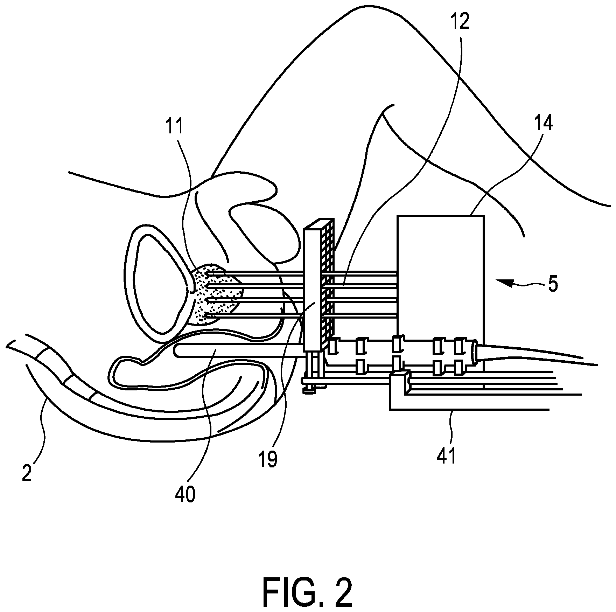

Fig. 1 shows schematically and exemplarily a system 1 for treating a subject 2 arranged on a patient table 3. The system 1 comprises a placing unit 5 for placing needles 12 within the subject 2, which is schematically and exemplarily shown in more detail in Fig. 2.

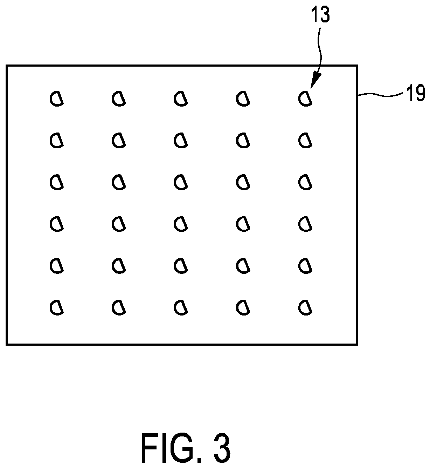

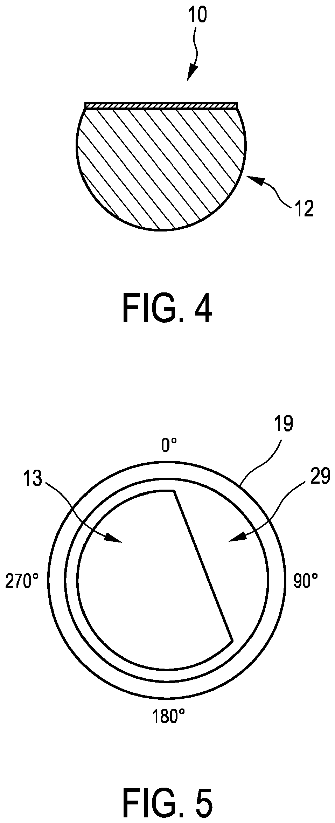

The placing unit 5 comprises a support structure 19 comprising several openings 13 arranged in a two-dimensional array in a holding plane for supporting the needles 12. The support structure 19, which can also be regarded as being a template, is schematically and exemplarily shown in more detail in Fig. 3, wherein Fig. 4 shows that the needles 12 have a non-circular cross section and an energy delivery element 10 with a flat surface being preferentially an HIFU element extending along a portion of the respective needle 12. Thus, the needles 12 are unidirectional treatment devices. Moreover, the needles 12 are interstitial treatment devices.

The openings 13 of the support structure 19 have a cross section which corresponds to the cross section of the needles 12. One of these openings 13 is schematically and exemplarily shown in more detail in Fig. 5. In Fig. 5 only a portion of the support structure 19 is shown, which surrounds a respective inner holding element 29, wherein the inner holding 29 is rotatable with respect to the support structure 19 and comprises the opening 13. The needles 12 can be inserted into the openings 13, wherein, after the needles 12 have been inserted into the openings 13, the needles 12 can perform a translational movement perpendicular to the holding plane defined by the support structure 19 and the needles 12 can be rotated around their respective longitudinal axis. If the support structure 19 is regarded as defining an x-y plane of a Cartesian coordinate system, the x-y position of the respective needle 12 can be chosen by choosing a respective opening 13 within the support structure 19 and the position of the respective needle 12 in a z direction being perpendicular to the x-y plane can be chosen by moving the respective needle 12 accordingly in the z direction through the chosen opening 13. Moreover, by rotating the respective needle 12 within the support structure 19 around its longitudinal axis the respective needle 12 can also angularly be rotated as desired. The support structure 19 therefore allows for a positioning of the needles 12 in a four-dimensional space represented by the three Cartesian coordinates x, y, z and the angular rotation around the longitudinal axis of the respective needle 12.

In this embodiment the needles 12 are moved in the z direction and rotated around their respective longitudinal axis by using a motor device 14. The motor device 14 is preferentially adapted to allow for an individual movement of each needle 12 such that each needle 12 can be moved as desired.

The needles 12 are placed within the prostate 11 of the subject 2, in order to treat a tumor within the prostate 11. The placing unit 5 further comprises an ultrasound data generating unit 40 being, in this embodiment, a transrectal ultrasound (TRUS) probe attached to a carrying element 41 to which also the support structure 19 and the motor device 14 are attached. The TRUS probe 40 is connected to an ultrasound image generating unit 42, which is located in a processing and control device 7, for generating an ultrasound image of the prostate.

The processing and control device 7 also comprises a placing control unit 15 for controlling the placing unit 5 depending on a determined treatment plan. In particular, the placing control unit 15 is adapted to control the motor device 14 and hence the positions of the needles 12 such that the treatment is performed in accordance with a determined treatment plan. The placing unit 5 and placing control unit 15 can be regarded as forming an arranging device for arranging the needles 12 in accordance with treatment positions defined by the treatment plan. The arranging device, especially the placing control unit 15, can be adapted to determine the current position of each needle 12 in the four-dimensional space based on information about how much each needle 12 has been rotated and translationally moved, which the placing control unit 15 may have already, because it controls the motor device 14, or which the placing control unit 15 may receive from the motor device 14. Moreover, the placing control unit 15 may be adapted to identify the needles 12 in an ultrasound image provided by the ultrasound image generating unit 42 by using, for instance, known segmentation algorithms. The ultrasound image can especially be used for verifying and optionally correcting the determined z positions of the needles 12. The placing control unit 15 is also adapted to control the energy to be delivered by the needles 12 via the energy delivery elements 10.

The system further comprises a treatment planning device 39 for planning the treatment of the subject, i.e. for determining a treatment plan. The treatment plan includes at least desired treatment positions of the needles 12 and the amount of energy to be delivered via the energy delivery elements 10. The treatment planning device 39 preferentially comprises relations between a) the amount of delivered energy and b) the shape and extension of an ablation region, wherein these relations are preferentially functional relations. However, they can also be non-functional and be stored in, for example, a lookup table. The treatment planning device 39 also comprises an image data set in which the tumor to be treated has been identified and in which preferentially also surrounding elements like organs, blood vessels, et cetera are identified. The treatment planning device 39 is preferentially adapted for determining at least treatment positions of the needles 12 and amounts of energy to be delivered by the energy delivery elements of the needles 12 such that the ablation regions completely cover the tumor to be treated and preferentially also a safety margin around the tumor and do substantially not cover surrounding parts of the subject 2 like healthy organ tissue, blood vessels, et cetera. The determined treatment positions and amounts of energy to be delivered are provided to the placing control unit 15 which controls the placing unit 5 accordingly.

The system 1 further comprises an input unit 30 like a keyboard, a computer mouse, a touchpad, et cetera, in order to allow a user to provide inputs into the system. Moreover, the system 1 comprises an output unit 31 like a display for displaying, for instance, the ultrasound image, a planned treatment position of one or several needles 12, a current position of one or several needles 12, the tumor, the ablation regions, et cetera.

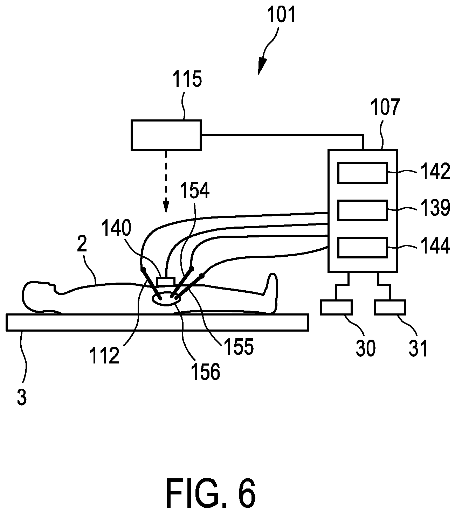

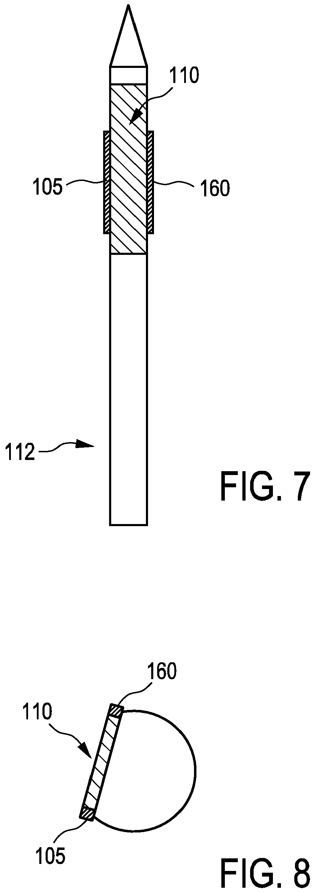

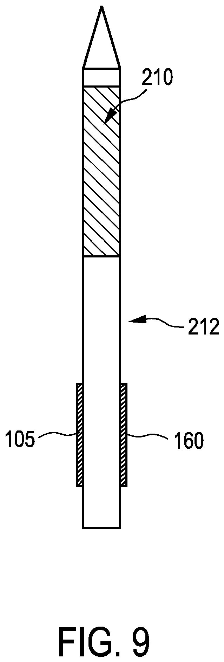

Fig. 6 shows schematically and exemplarily a further embodiment of a system 101 for treating a subject. In this embodiment the system 101 comprises several needles 112, 154, 155, wherein one of these needles is schematically and exemplarily shown in more detail in Fig. 7. The needles 112, 154, 155 can be handheld needles or they can be needles to be automatically positioned by using, for instance, a robotic device. The needles comprise an energy delivery element 110 and a six-degrees-of-freedom sensor arranged close to the energy delivery element 110. In this embodiment the six-degrees-of-freedom sensor comprises two electromagnetic location sensors 105, 160 arranged at opposing sides of the energy delivery element 110. Fig. 8 shows schematically and exemplarily a cross sectional view of the needle shown in Fig. 7. As can be seen in this Fig. 8, also the needles used in this embodiment have a non-circular cross section, wherein the energy delivery element 110 comprises a flat surface emitting the energy. Moreover, also in this embodiment the energy delivery element 110 is a HIFU element. In other embodiments the electromagnetic sensors may be arranged in another way. For instance, as schematically and exemplarily shown in Fig. 9, two electromagnetic sensors 105, 160 of a needle 212 may be arranged with a distance to the energy delivery element 210, wherein this distance might be, for instance, 3 cm or more, 5 cm or more, or 10 cm or more.

The electromagnetic sensors 105, 160 are used together with an electromagnetic tracking unit 115 for determining the positions of the needles 112, 154, 155 and hence of the respective energy delivery elements 110 in a four-dimensional space which is representable by three Cartesian coordinates and an angular coordinate, wherein the angular coordinate defines the angular orientation of the respective needle 112, 154, 155 with respect to a rotation around the longitudinal axis of the respective needle 112, 154, 155. The determined positions of the needles 112, 154, 155 are shown on a display 31 together with planned treatment positions of the needles 112, 154, 155, in order to assist a user in placing the needles 112, 154, 155 in accordance with the planned treatment positions. If a robotic device is used for positioning the needles 112, 154, 155, the determined current positions of the needles 112, 154, 155 can also be provided to the robotic device, in order to allow the robotic device to steer the needles 112, 154, 155 based on their current positions and their desired planned treatment positions.

The system 101 further comprises an ultrasound sensor 140 connected to an ultrasound image generation unit 142 of a processing and control device 107 for generating an ultrasound image of the tumor and the needles 112, 154, 155, wherein the tumor may be a tumor within an organ 156 like the liver or another organ.

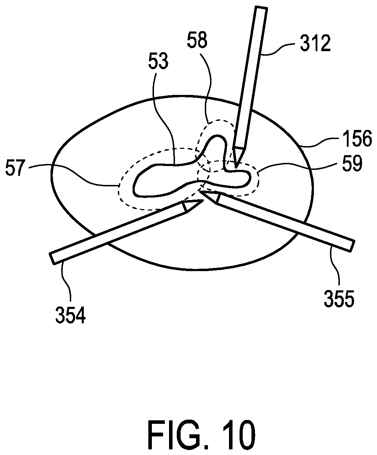

Also the system 101 comprises a treatment planning device. The treatment planning device 139 of the present embodiment is similar to the treatment planning device 39 described above with reference to Fig. 1. In particular, also in this embodiment relations between a) ablation regions generated by the needles 112, 154, 155 and b) amounts of energy to be delivered are used together with an image data set showing the tumor within the organ 156 and possibly further parts of the subject 2, which should not be adversely affected by the treatment, for determining the treatment plan. The treatment planning device 139 determines treatment positions of the needles 112, 155, 156 and amounts of energy to be delivered by the energy delivery elements of the needles such that the ablation regions cover the tumor and preferentially also a safety margin around the tumor and not or as less as possible healthy tissue surrounding the safety margin. The result of such a treatment planning is schematically and exemplarily illustrated in Fig. 10.

Fig. 10 illustrates the treatment positions 312, 354, 355 of the needles 112, 154, 155 and the ablation regions 57, 58, 59, which will be obtained, when energy is delivered in accordance with the treatment plan. As can be seen in Fig. 10, the ablation regions 57, 58, 59 cover the tumor 53 and a safety margin around the tumor 53, but no further healthy tissue of the organ 156. It should be noted that the treatment planning also includes the planning of the angular orientation of the respective needle with respect to a rotation of the respective needle around its longitudinal axis, although the respective angular orientation is not illustrated in Fig. 10.

After the treatment plan has been generated and the needles 112, 154, 155 have been arranged in accordance with the determined planed treatment positions, an energy delivery control unit 144 controls the energy delivered by the energy delivery elements 110 of the needles 112, 154, 155 in accordance with the planned amounts of energy. The energy delivery control unit 144 can be adapted to control the energy delivery elements such that they deliver the energy only, if a deviation between the planned treatment positions of the needles 112, 154, 155 and the determined current positions of the needles 112, 154, 155 is smaller than a predefined threshold. This can ensure that the energy is only delivered, if the needles 112, 154, 155 are accurately positioned.

Also in this embodiment the system 101 comprises an input unit 30 like a keyboard, a computer mouse, a touchpad, et cetera and an output unit 31 like a display. The display 31 may show, for instance, an ultrasound image, determined current positions of the needles, planned treatment positions of the needles, the ablation regions and the tumor as illustrated in Fig. 10, et cetera.

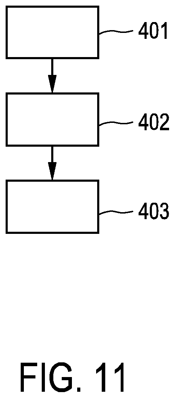

In the following an embodiment of a method for treating a subject will exemplarily be described with reference to a flowchart shown in Fig. 11.

In step 401 a treatment of the subject is planned by using a treatment planning device. In particular, a treatment position of a treatment device to be used during the treatment is determined in a four-dimensional space, wherein the four-dimensional space is representable by three Cartesian coordinates and an angular coordinate, wherein the angular coordinate defines the angular orientation of the treatment device with respect to a rotation around its longitudinal axis. Moreover, in step 401 the amount of energy to be delivered by an energy delivery element of the treatment device during the treatment is determined. This step can also be regarded as being a step of a method for planning a treatment of a subject. In step 402 the treatment device is arranged in accordance with the treatment position and in step 403 the energy is delivered as planned by the treatment planning device. This step can also be regarded as being a step of a method for arranging a treatment device.

The system and method for treating a subject can be adapted to use interstitial unidirectional energy delivery devices, i.e. treatment devices, for focal therapy which uses elevated temperatures for ablating focal lesions. The unidirectional treatment devices provide a confined and directional tissue heating for treating unifocal and multifocal cancer lesions. Thermal energy clouds, i.e. ablation regions, generated by the energy delivery elements of the treatment devices can be directed on top of a to-be-treated volume, wherein the treatment devices can be steered such that the to-be-treated volume and optionally also a safety margin around this volume is completely covered by the thermal energy clouds generated by the energy delivery elements of the treatment devices. In an embodiment the treatment devices have each a width of up to 2 mm. However, the width of the treatment devices can also be larger or smaller. The heating system, i.e. the energy delivery element, of the respective treatment device can have the same width and can have a length of, for instance, few centimeters. In particular, the length of the respective energy delivery element can be chosen depending on the size of the lesion to be treated.

The treatment planning device can be adapted to not only determine the positions and amounts of the energy to be delivered during the treatment, but also the number of treatment devices and/or the kind of treatment devices, in particular, the lengths of the energy delivery elements of the treatment devices. After the treatment planning device has determined a certain number of treatment devices having the same or different certain energy delivery element lengths, the user or, for instance, a robotic device can arrange a corresponding number of treatment devices having the planned energy delivery element lengths within the subject in accordance with the planned treatment positions of the treatment devices. Several treatment devices can be used synergistically for ablating a lesion as illustrated, for instance, in Fig. 10.

The determination of the treatment positions and the following arrangement procedure in accordance with the determined treatment positions is four-dimensional, i.e. a geometric x, y, z position and an angular orientation are considered, wherein the angular orientation determines which part of the tissue around the respective treatment device is heated. Each treatment device can be a needle comprising a flat energy delivery element, wherein this combination of a needle with the flat energy delivery element preferentially results in a treatment device having a rounded side and a flat side as shown, for instance, in Fig. 4. For positioning the treatment devices and hence the energy delivery elements in the Cartesian coordinate system defined by the coordinates x, y, z a support structure having a two-dimensional array of openings as schematically and exemplarily shown in, for instance, Figs. 2 and 3 can be used, wherein for checking the z positions of the treatment devices an ultrasound imaging unit, especially the TRUS imaging unit described above with reference to Fig. 2, may be used. Also for checking the x, y positions of the treatment devices an ultrasound image may be used. The openings in the support structure may be formed by an inner part which is separately rotatable with respect to the support structure as described above with reference to Fig. 5, in order to set the rotation angle. This rotation angle may be set manually, wherein an angular scale may be present on the support structure and/or on the rotatable inner part, or the rotation may be motorized. For instance, the motor device 14 described above with reference to Fig. 2 may be used. However, it is also possible that another motor device is used. For instance, each opening of the support structure can comprise a motor for rotating the respective inner part relative to the support structure. The rotation angle is preferentially set in accordance with the respective rotation angle planned by the treatment planning device.

For checking the orientation of a treatment device a six-degrees-of-freedom electromagnetic sensor may be used, for instance, as described above with reference to Figs. 7 to 9. The six-degrees-of-freedom electromagnetic sensors or other six-degrees-of-freedom sensors can also be used in combination with other treatment devices, in particular, in combination with the needles 12 described above with reference to Figs. 1 to 5.

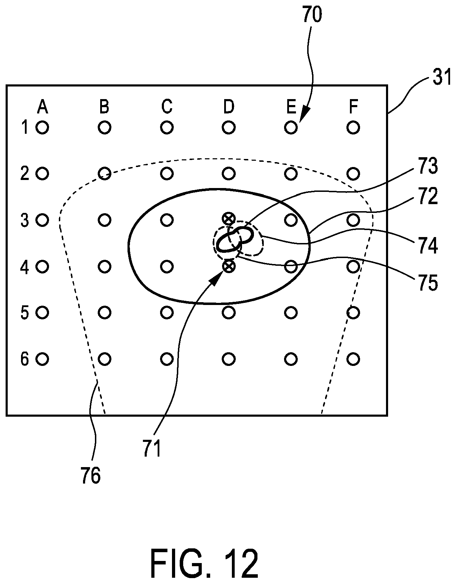

If the support structure 19 with the two-dimensional array of openings 13 is used for arranging the treatment devices 12 in accordance with the treatment plan, the treatment planning device 39 is preferentially adapted to output in which of the openings 13 a treatment device 12 should be inserted and how deep it should be inserted into the subject, i.e., for instance, how large the distance between the distal tip of the respective needle 12 and the support structure 19 should be in the z direction, thereby defining the x, y, z coordinates of the treatment position. The determined openings of the support structure 19, through which the treatment devices 12 should be inserted into the subject, can be indicated on an image of the grid of openings of the support structure, which can be shown on the display 31. As an overlay on the image of the grid of openings of the support structure in addition at least one of a) an ultrasound image of the subject, b) one or several ablation regions, c) a tumor to be treated, d) an organ in which the tumor may be located, et cetera may be shown on the display 31. For instance, as schematically illustrated in Fig. 12, the display 31 may show openings 70 of the support structure, wherein some openings 71 are emphasized for indicating that the treatment devices should be inserted into these openings 71. Moreover, as an overlay an organ 72, a tumor 73 within the organ 72, ablation regions 74, 75, which will be generated by the treatment devices, if they are operated in accordance with the treatment plan, and an ultrasound image as indicated by the broken lines 76 may be shown. The display 31 may also indicate the planned angular orientations of the treatment devices 12 with respect to their longitudinal axes.

Although in above described embodiments the treatment devices comprise HIFU elements as energy delivery elements, in other embodiments other energy delivery elements can be used like electrodes delivering electrical energy or outcoupling regions of optical fibers for delivering optical energy. It is also possible to use ultrasound elements which are not HIFU elements. For instance, a plane high intensity ultrasound element may be used, of which the focus point is a natural focus. Moreover, although in above described embodiments several treatment devices are used for the treatment, in other embodiments also a single treatment device may be used for the treatment.

Although the treatment devices described above with reference to Figs. 4 and 7 to 9 have a cross section with a flat side and a rounded side substantially along the entire length of the respective treatment device, in other embodiments the treatment devices may have such a cross section only in a region in which the energy delivery element is arranged. It is also possible that the treatment devices have another cross section along their entire length like a circular cross section.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

The treatment planning device and the arranging device can be integrated in a same device or they can be separate devices.

Procedures like the determination of the treatment plan performed by one or several units or devices can be performed by any other number of units or devices. These procedures and/or the control of the system for treating a subject in accordance with the method for treating a subject and/or the control of the treatment planning device in accordance with the method for planning a treatment of a subject and/or the control of the arranging device in accordance with the method for arranging a treatment device can be implemented as program code means of a computer program and/or as dedicated hardware. In particular, the treatment planning device and/or the arranging device can be formed by means of a computer program running on a computing device and/or by dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for planning a treatment of a subject. A treatment planning device determines a treatment position of a treatment device like a needle or catheter which comprises an energy delivery element not completely encircling the treatment device. The determined treatment position is a position in a four-dimensional space being representable by three Cartesian coordinates and an angular coordinate defining the angular orientation of the treatment device with respect to a rotation around its longitudinal axis. The treatment device is then arranged in accordance with the treatment position. Since not only the three-dimensional position of the treatment device is determined, but also a rotational position of the treatment device with respect to a rotation of the treatment device around its longitudinal axis, the energy delivery can be very accurately planned, leading to a reduction of unwanted therapy side effects.

The present invention is set out in the appended claims. The embodiments, aspects or examples according to the present description that do not fall within the scope of said claims are provided for illustrative purposes only and do not form part of the present invention.

## Claims

1. A system for planning a treatment of a subject (2), the system (1; 101) being adapted to plan a treatment to be carried out by using a treatment device (12; 112, 154, 155, 212) suitable for being placed within the subject, the treatment device (12; 112, 154, 155, 212) being elongated and comprising a longitudinal axis and an energy delivery element (10; 110; 210) which does not completely encircle the treatment device (12; 112, 154, 155, 212) and which is adapted to deliver energy to be used for treating the subject (2), wherein the system (1) further comprises:
a treatment planning device (39; 139) for planning the treatment of the subject, wherein the treatment planning device (39; 139) is adapted to determine a treatment position of the treatment device (12; 112, 154, 155, 212) to be used during the treatment in a four-dimensional space, wherein the four-dimensional space is representable by three Cartesian coordinates and an angular coordinate, wherein the angular coordinate defines the angular orientation of the treatment device (12; 112, 154, 155, 212) with respect to a rotation around its longitudinal axis.

2. The system as defined in claim 1, wherein the system further comprises an arranging device (5, 15; 105, 115, 160) for receiving the treatment position determined by the treatment planning device (39; 13) and for arranging the treatment device (12; 112, 154, 155, 212) in accordance with the received treatment position.

3. The system as defined in claim 2, wherein the arranging device (5, 15) includes a support structure (19) comprising at least one opening (13) for receiving the treating device (12), wherein the support structure (19) is adapted such that the treatment device (12) is rotatable relative to the support structure (19), in order to arrange the treatment device (12) in accordance with the angular coordinate of the treatment position.

4. The system as defined in claim 3, wherein the support structure (19) comprises at least one holding element (29) comprising the at least one opening (13) for receiving the treatment device (12), wherein the holding element (29) is rotatable relative to the support structure (19), in order to arrange the treatment device (12) in accordance with the angular coordinate of the treatment position.

5. The system as defined in claim 3, wherein the at least one opening (13) has a non-circular cross section.

6. The system as defined in claim 3, wherein the support structure (19) comprises several openings (13) for receiving the treatment device (12), wherein the openings (13) are arranged in a holding plane and are adapted such that the treatment device (12) is movable in a direction being perpendicular to the holding plane.

7. The system as defined in claim 2, wherein the arranging device (5, 15; 105, 115, 160) is adapted to determine the current position of the treatment device (12; 112, 154, 155, 212) in the four-dimensional space and to arrange the treatment device (12; 112, 154, 155, 212) based on a difference between the current position and the treatment position and/or to output the difference.

8. The system as defined in claim 7, wherein the arranging device (105, 115, 160) comprises a six-degrees-of-freedom sensor (105, 160) for determining the position of the treatment device (112, 154, 155).

9. The system as defined in claim 8, wherein the six-degrees-of-freedom sensor (105, 160) is an electromagnetic sensor.

10. The system as defined in claim 8, wherein the six-degrees-of-freedom sensor (105, 160) comprises at least two location sensors arranged at opposite sides of the energy delivery element (110).

11. The system as defined in claim 8, wherein the six-degrees-of-freedom sensor (105, 160) comprises at least two location sensors arranged with a distance to the energy delivery element (210).

12. The system as defined in claim 1, wherein the system comprises the treatment device (12; 112, 154, 155, 212) and wherein the treatment device (12; 112, 154, 155, 212) is a unidirectional treatment device (12; 112, 154, 155, 212).

13. An arranging device for arranging a treatment device (12; 112, 154, 155, 212), the treatment device (12; 112, 154, 155, 212) being elongated and comprising a longitudinal axis and an energy delivery element (10; 110; 210) which does not completely encircle the treatment device (12; 112, 154, 155, 212) and which is adapted to deliver energy to be used for treating the subject (2), **characterized in that** the arranging device is adapted for receiving a treatment position determined by a treatment planning device (39; 13) of a system as defined in claim 1 and for arranging the treatment device (12; 112, 154, 155, 212) in accordance with the received treatment position.

14. A method for planning a treatment of a subject (2), the method being adapted to plan a treatment to be carried out by using a treatment device (12; 112, 154, 155 212) suitable for being placed within a subject, the treatment device (12; 112, 154, 155, 212) being elongated and comprising a longitudinal axis and an energy delivery element (10) which does not completely encircle the treatment device (12; 112, 154, 155, 212) and which is adapted to deliver energy to be used for treating the subject (2), wherein the method further comprises:
planning the treatment of the subject (2) by using a treatment planning device (39; 139), wherein a treatment position of the treatment device (12; 112, 154, 155, 212) to be used during the treatment is determined in a four-dimensional space, wherein the four-dimensional space is representable by three Cartesian coordinates and an angular coordinate, wherein the angular coordinate defines the angular orientation of the treatment device (12; 112, 154, 155, 212) with respect to a rotation around its longitudinal axis.

15. A computer program for controlling a system for planning a treatment of a subject (2) as defined in claim 1, **characterized in that** the computer program comprises program code means for causing the system to carry out the steps of the method for planning a treatment of a subject (2) as defined in claim 14, when the computer program is run on a computer controlling the system.

## Patentansprüche

1. System zur Planung einer Behandlung eines Subjekts (2), wobei das System (1; 101) angepasst ist, um eine Behandlung, die auszuführen ist, zu planen, indem eine Behandlungsvorrichtung (12; 112, 154, 155, 212) verwendet wird, die geeignet ist, innerhalb des Subjekts platziert zu sein,
wobei die Behandlungsvorrichtung (12; 112, 154, 155, 212) länglich ist und eine Längsachse und ein Energielieferelement (10; 110; 210), das die Behandlungsvorrichtung (12; 112, 154, 155, 212) nicht vollständig einkreist, umfasst, und das angepasst ist, um Energie zu liefern, die zur Behandlung des Subjekts (2) zu verwenden ist, wobei das System (1) weiter Folgendes umfasst:
eine Behandlungsplanungsvorrichtung (39; 139) zur Planung der Behandlung des Subjekts, wobei die Behandlungsplanungsvorrichtung (39; 139) angepasst ist, um eine Behandlungsposition der Behandlungsvorrichtung (12; 112, 154, 155, 212), die während der Behandlung zu verwenden ist, in einem vierdimensionalen Raum zu bestimmen, wobei der vierdimensionale Raum durch drei kartesische Koordinaten und eine Winkelkoordinate darstellbar ist, wobei die Winkelkoordinate die Winkelausrichtung der Behandlungsvorrichtung (12; 112, 154, 155, 212) in Bezug auf eine Drehung um ihre Längsachse definiert.

2. System nach Anspruch 1, wobei das System weiter eine Einrichtungsvorrichtung (5, 15; 105, 115, 160) umfasst, um die Behandlungsposition zu empfangen, die von der Behandlungsplanungsvorrichtung (39; 13) bestimmt wird, und um die Behandlungsvorrichtung (12; 112, 154, 155, 212) in Übereinstimmung mit der empfangenen Behandlungsposition einzurichten.

3. System nach Anspruch 2, wobei die Einrichtungsvorrichtung (5, 15) eine Tragstruktur (19) beinhaltet, die mindestens eine Öffnung (13) zum Empfangen der Behandlungsvorrichtung (12) umfasst, wobei die Tragstruktur (19) derart angepasst ist, dass die Behandlungsvorrichtung (12) in Bezug auf die Tragstruktur (19) drehbar ist, um die Behandlungsvorrichtung (12) in Übereinstimmung mit der Winkelkoordinate der Behandlungsposition einzurichten.

4. System nach Anspruch 3, wobei die Tragstruktur (19) mindestens ein Halteelement (29) umfasst, das die mindestens eine Öffnung (13) zum Aufnehmen der Behandlungsvorrichtung (12) umfasst, wobei das Halteelement (29) in Bezug auf die Tragstruktur (19) drehbar ist, um die Behandlungsvorrichtung (12) in Übereinstimmung mit der Winkelkoordinate der Behandlungsposition einzurichten.

5. System nach Anspruch 3, wobei die mindestens eine Öffnung (13) einen nicht kreisförmigen Querschnitt aufweist.

6. System nach Anspruch 3, wobei die Tragstruktur (19) mehrere Öffnungen (13) zum Aufnehmen der Behandlungsvorrichtung (12) umfasst, wobei die Öffnungen (13) in einer Halteebene eingerichtet und derart angepasst sind, dass die Behandlungsvorrichtung (12) in eine Richtung, die zu der Halteebene senkrecht ist, bewegbar ist.

7. System nach Anspruch 2, wobei die Einrichtungsvorrichtung (5, 15; 105, 115, 160) angepasst ist, um die aktuelle Position der Behandlungsvorrichtung (12; 112, 154, 155, 212) in dem vierdimensionalen Raum zu bestimmen und die Behandlungsvorrichtung (12; 112, 154, 155, 212) basierend auf einem Unterschied zwischen der aktuellen Position und der Behandlungsposition einzurichten und/oder den Unterschied auszugeben.

8. System nach Anspruch 7, wobei die Einrichtungsvorrichtung (105, 115, 160) einen Sensor mit sechs Freiheitsgraden (105, 160) zum Bestimmen der Position der Behandlungsvorrichtung (112, 154, 155) umfasst.

9. System nach Anspruch 8, wobei der Sensor mit sechs Freiheitsgraden (105, 160) ein elektromagnetischer Sensor ist.

10. System nach Anspruch 8, wobei der Sensor mit sechs Freiheitsgraden (105, 160) mindestens zwei Lagesensoren umfasst, die an entgegengesetzten Seiten des Energielieferelements (110) eingerichtet sind.

11. System nach Anspruch 8, wobei der Sensor mit sechs Freiheitsgraden (105, 160) mindestens zwei Lagesensoren umfasst, die in einem Abstand von dem Energielieferelement (210) eingerichtet sind.

12. System nach Anspruch 1, wobei das System die Behandlungsvorrichtung (12; 112, 154, 155, 212) umfasst, und wobei die Behandlungsvorrichtung (12; 112, 154, 155, 212) eine Einrichtungsbehandlungsvorrichtung (12; 112, 154, 155, 212) ist.

13. Einrichtungsvorrichtung für das Einrichten einer Behandlungsvorrichtung (12; 112, 154, 155, 212), wobei die Behandlungsvorrichtung (12; 112, 154, 155, 212) länglich ist und eine Längsachse und ein Energielieferelement (10; 110; 210), das die Behandlungsvorrichtung (12; 112, 154, 155, 212) nicht vollständig einkreist, umfasst, und das angepasst ist, um Energie zu liefern, die zum Behandeln des Subjekts (2) zu verwenden ist, **dadurch gekennzeichnet, dass** die Einrichtungsvorrichtung angepasst ist, um eine Behandlungsposition zu empfangen, die von einer Behandlungsplanungsvorrichtung (39; 13) eines Systems nach Anspruch 1 bestimmt wird, und um die Behandlungsvorrichtung (12; 112, 154, 155, 212) in Übereinstimmung mit der empfangenen Behandlungsposition einzurichten.

14. Verfahren zur Planung einer Behandlung eines Subjekts (2), wobei das Verfahren angepasst ist, um eine Behandlung zu planen, die unter Verwenden einer Behandlungsvorrichtung (12; 112, 154, 155 212), die geeignet ist, um innerhalb eines Subjekts platziert zu werden, auszuführen ist, wobei die Behandlungsvorrichtung (12; 112, 154, 155, 212) länglich ist und eine Längsachse und ein Energielieferelement (10), das die Behandlungsvorrichtung (12; 112, 154, 155, 212) nicht vollständig einkreist, umfasst, und das angepasst ist, um Energie zu liefern, die zum Behandeln des Subjekts (2) angepasst ist, wobei das Verfahren weiter Folgendes umfasst:
Planen der Behandlung des Subjekts (2) durch Verwenden einer Behandlungsplanungsvorrichtung (39; 139), wobei eine Behandlungsposition der Behandlungsvorrichtung die Behandlungsplanungsvorrichtung (12; 112, 154, 155, 212), die während der Behandlung zu verwenden ist, in einem vierdimensionalen Raum bestimmt wird, wobei der vierdimensionale Raum durch drei kartesische Koordinaten und eine Winkelkoordinate darstellbar ist, wobei die Winkelkoordinate die Winkelausrichtung der Behandlungsvorrichtung (12; 112, 154, 155, 212) in Bezug auf eine Drehung um ihre Längsachse definiert.

15. Computerprogramm zum Steuern eines Systems zum Planen einer Behandlung eines Subjekts (2) nach Anspruch 1, **dadurch gekennzeichnet, dass** das Computerprogramm Programmcodemittel umfasst, um das System zu veranlassen, die Schritte des Verfahrens zur Planung einer Behandlung eines Subjekts (2), wie in Anspruch 14 definiert, auszuführen, wenn das Computerprogramm auf einem Computer, der das System steuert, ausgeführt wird.

## Revendications

1. Système de planification d'un traitement d'un sujet (2), le système (1 ; 101) étant à même de planifier un traitement à effectuer en utilisant un dispositif de traitement (12 ; 112, 154,155, 212) convenant pour être placé à l'intérieur du sujet,
le dispositif de traitement (12 ; 112, 154, 155, 212) étant allongé et comprenant un axe longitudinal et un élément de fourniture d'énergie (10 ; 110 ; 210) qui n'entoure par complètement le dispositif de traitement (12 ; 112, 154, 155, 212) et qui est à même de fournir de l'énergie à utiliser pour traiter le sujet (2), dans lequel le système (1) comprend en outre :
un dispositif de planification de traitement (39 ; 139) afin de planifier le traitement du sujet, dans lequel le dispositif de planification de traitement (39 ; 139) est à même de déterminer une position de traitement du dispositif de traitement (12 ; 112, 154, 155, 212) à utiliser au cours du traitement dans un espace à quatre dimensions, dans lequel l'espace à quatre dimensions peut être représenté par trois coordonnées cartésiennes et une coordonnée angulaire, dans lequel la coordonnée angulaire définit l'orientation angulaire du dispositif de traitement (12 ; 112, 154, 155, 212) par rapport à une rotation autour de son axe longitudinal.

2. Système selon la revendication 1, dans lequel le système comprend en outre un dispositif d'agencement (5, 15 ; 105, 115, 160) pour recevoir la position de traitement déterminée par le dispositif de planification de traitement (39 ; 13) et pour agencer le dispositif de traitement (12 ; 112, 154, 155, 212) conformément à la position de traitement reçue.

3. Système selon la revendication 2, dans lequel le dispositif d'agencement (5, 15) comprend une structure de support (19) comportant au moins une ouverture (13) afin de recevoir le dispositif de traitement (12), dans lequel la structure de support (19) est adaptée de sorte que le dispositif de traitement (12) puisse tourner par rapport à la structure de support (19) en sorte d'agencer le dispositif de traitement (12) conformément à la coordonnée angulaire de la position de traitement.

4. Système selon la revendication 3, dans lequel la structure de support (19) comprend au moins un élément de retenue (29) comprenant la au moins une ouverture (13) pour recevoir le dispositif de traitement (12), dans lequel l'élément de retenue (29) peut tourner par rapport à la structure de support (19) en sorte d'agencer le dispositif de traitement (12) conformément à la coordonnée angulaire de la position de traitement.

5. Système selon la revendication 3, dans lequel la au moins une ouverture (13) a une section transversale non circulaire.

6. Système selon la revendication 3, dans lequel la structure de support (19) comprend plusieurs ouvertures (13) pour recevoir le dispositif de traitement (12), dans lequel les ouvertures (13) sont agencées dans un plan de retenue et sont adaptées de sorte que le dispositif de traitement (12) soit mobile dans une direction qui est perpendiculaire au plan de retenue.

7. Système selon la revendication 2, dans lequel le dispositif d'agencement (5, 15 ; 105, 115, 160) est à même de déterminer la position courante du dispositif de traitement (12 ; 112, 154, 155, 212) dans l'espace à quatre dimensions et d'agencer le dispositif de traitement (12 ; 112, 154, 155, 212) sur la base d'une différence entre la position courante et la position de traitement et/ou de délivrer la différence.

8. Système selon la revendication 7, dans lequel le dispositif d'agencement (105, 115, 160) comprend un capteur à six degrés de liberté (105, 160) pour déterminer la position du dispositif de traitement (112, 154, 155).

9. Système selon la revendication 8, dans lequel le capteur à six degrés de liberté (105, 160) est un capteur électromagnétique.

10. Système selon la revendication 8, dans lequel le capteur à six degrés de liberté (105, 160) comprend au moins deux capteurs de localisation agencés sur les côtés opposés de l'élément de fourniture d'énergie (110).

11. Système selon la revendication 8, dans lequel le capteur à six degrés de liberté (105, 160) comprend au moins deux capteurs de localisation agencés à une certaine distance de l'élément de fourniture d'énergie (210).

12. Système selon la revendication 1, dans lequel le système comprend le dispositif de traitement (12 ; 112, 154, 155, 212) et dans lequel le dispositif de traitement (12 ; 112, 154, 155, 212) est un dispositif de traitement unidirectionnel (12 ; 112, 154, 155, 212).

13. Dispositif d'agencement pour agencer un dispositif de traitement (12 ; 112, 154, 155, 212), le dispositif de traitement (12 ; 112, 154, 155, 212) étant allongé et comprenant un axe longitudinal et un élément de fourniture d'énergie (10 ; 110 ; 210) qui n'entoure pas complètement le dispositif de traitement (12 ; 112, 154, 155, 212) et qui est à même de fournir de l'énergie à utiliser pour traiter le sujet (2), **caractérisé en ce que** le dispositif d'agencement est à même de recevoir une position de traitement déterminée par un dispositif de planification de traitement (39 ; 13) d'un système selon la revendication 1 et d'agencer le dispositif de traitement (12 ; 112, 154, 155, 212) conformément à la position de traitement reçue.

14. Procédé de planification d'un traitement d'un sujet (2), le procédé étant à même de planifier un traitement à effectuer en utilisant un dispositif de traitement (12 ; 112, 154, 155, 212) convenant pour être placé à l'intérieur d'un sujet, le dispositif de traitement (12 ; 112, 154, 155, 212) étant allongé et comprenant un axe longitudinal et un élément de fourniture d'énergie (10) qui n'entoure pas complètement le dispositif de traitement (12 ; 112, 154, 155, 212) et qui est à même de fournir de l'énergie à utiliser pour le traitement du sujet (2), dans lequel le procédé comprend en outre :
la planification du traitement du sujet (2) en utilisant un dispositif de planification de traitement (39 ; 139), dans lequel une position de traitement du dispositif de traitement (12 ; 112, 154, 155, 212) à utiliser au cours du traitement est déterminée dans un espace à quatre dimensions, dans lequel l'espace à quatre dimensions peut être représenté par trois coordonnées cartésiennes et une coordonnée angulaire, dans lequel la coordonnée angulaire définit l'orientation angulaire du dispositif de traitement (12 ; 112, 154, 155, 212) par rapport à une rotation autour de son axe longitudinal.

15. Programme d'ordinateur pour commander un système de planification d'un traitement d'un sujet (2) selon la revendication 1, **caractérisé en ce que** le programme d'ordinateur comprend des moyens de codage de programme pour amener le système à effectuer les étapes de planification d'un traitement d'un sujet (2) selon la revendication 14, lorsque le programme d'ordinateur tourne sur un ordinateur commandant le système.
